# EUROPÄISCHE PATENTANMELDUNG

(11) **EP 4 197 470 A1**
(43) Veröffentlichungstag der Anmeldung: **21.06.2023**
(21) Anmeldenummer: 21215672.3
(22) Anmeldetag: 17.12.2021
(51) Int. Cl.: A61B 18/14

(54) **ELEKTROCHIRURGISCHES INSTRUMENT**

(71) Anmelder: Erbe Elektromedizin GmbH, 72072 Tübingen (DE)
(72) Erfinder: Mueller, Marc, 72202 Nagold (DE); Kleber, Frederik, 72827 Wannweil (DE); Brodbeck, Achim, 72555 Metzingen (DE)
(74) Vertreter: Rüger Abel Patentanwälte PartGmbB

(57) **Zusammenfassung**

Das erfindungsgemäße Instrument (10) weist eine Haube (25) auf, die einen Strömungsraum (26) umschließt. Dieser dient der Absaugung von Emissionen, die durch Einwirkung einer hochfrequenzspannungsbeaufschlagten Elektrode (18) auf biologisches Gewebe entstehen. Zur Beseitigung von Gewebeniederschlag und sonstigem Verstopfungen aus den Strömungsraum (26) wird dieser von einer beweglichen Haube (25) begrenzt, die zu Reinigungszwecken aus ihrer Arbeitsposition heraus in eine Reinigungsposition überführbar ist.

## Beschreibung

Die Erfindung betrifft ein elektrochirurgisches Instrument zur Behandlung menschlicher oder tierischer Patienten. Insbesondere betrifft die Erfindung ein solches Instrument, das zusätzlich zu einer zur chirurgischen Behandlung eines Gewebes vorgesehenen Elektrode ein optisches Element zur Lichtaufnahme oder Lichtabgabe aufweist.

Aus der EP 2 659 846 A1 ist ein elektrochirurgisches Instrument mit einer Elektrode bekannt, die mittels elektrischen Stroms auf biologisches Gewebe einwirkt. An dem Instrument sind außerdem ein oder mehrere Lichtaufnahmeeinrichtungen vorgesehen, die Licht aufnehmen, das von einem an der Elektrode erzeugten Funkten ausgeht. Dieses Licht wird einer Lichtanalyseeinrichtung zugeführt, um daraus Gewebemerkmale zu ermitteln.

Ein ähnliches Instrument ist aus der EP 2 815 695 A1 bekannt. Das von dem jeweiligen Funken ausgehende Licht muss von den optischen Elementen des Instruments möglichst unverfälscht aufgenommen werden. Auf den optischen Elementen vorhandener Niederschlag, Gewebefragmente und dergleichen, stören dies.

Dazu schlägt die EP 2 815 713 B1 die Unterbringung eines lichtaufnehmenden Lichtleiters in einem inneren Fluidkanal eines Instruments vor. Dieses weist Fluidaustrittsöffnungen auf, über die Licht in den Fluidkanal eindringen kann. In der der Nähe der Lichtfluidaustrittsöffnungen ist eine Elektrode vorgesehen, die zur Gewebebehandlung dient.

Weiter ist aus der US 6,126,653 A ein Instrument mit einem in einem Rohr axial verschiebbaren Lichtleiter bekannt. An dem distalen Ende des Rohrs ist ein Bürstensatz angeordnet. Beim Vorschieben des Lichtleiters in distaler Richtung streift der Lichtleiter durch die Bürsten. Beim Rückziehen des Lichtleiters streifen die Bürsten auf dem Lichtleiter niedergeschlagene Partikel ab.

Ein ähnliches Konzept mit beweglichem Lichtleiter verfolgt die WO 2016/177600 A1. Das Instrument weist Reinigungsbürsten auf, die einen axial beweglichen Lichtleiter an seinem Umfang berühren.

Dagegen sieht die EP 1 210 904 A2 ein Instrument mit einem Außenrohr und einem darin axial beweglich gelagerten Lichtleiter auf. Das Außenrohr weist an dem distalen Ende eine schwenkbare Kappe mit einer Reinigungslippe auf. Wird der Lichtleiter in Distalrichtung axial vorgeschoben, schwenkt er durch seine Bewegung die Endkappe zur Seite. Zugleich streift die Reinigungslippe dieser Kappe über das stirnseitige Ende des Lichtleiters und reinigt diesen somit.

Die EP 2 862 533 B1 offenbart ein elektrochirurgisches Instrument mit einer Absaugeinrichtung. Diese umfasst einen Fluidkanal mit einer endseitigen Haube, die eine Ansaugöffnung umgibt. Die Haube kann mit der Elektrode fest verbunden und an dem Instrument abnehmbar angeordnet sein.

In der Praxis kann nicht immer ausgeschlossen werden, dass zum Reinigen von Lichtübertrittsfenstern vorgesehene Elemente, wie Bürsten oder dergleichen, selbst verschmutzen.

Es ist Aufgabe der Erfindung, ein Konzept anzugeben, mit dem elektrochirurgische Instrumente mit optischen Elementen auf lange Zeit betriebsfähig gehalten werden können.

Diese Aufgabe wird mit dem Instrument nach Anspruch 1 gelöst:
Das erfindungsgemäße Instrument kann ein für den offenchirurgischen Gebrauch vorgesehenes Instrument, ein für den laparoskopischen Gebrauch vorgesehenes Instrument, eine endoskopisch zu verwendende Sonde oder dergleichen sein. In jedem Fall weist das Instrument einen Instrumentenkörper auf, an dessen distalen Ende eine Elektrode zur Behandlung von biologischem Gewebe vorgesehen ist. Alternativ kann die Elektrode an einem mit dem Instrumentenkörper befestigten Element vorgesehen. Ein solches Element kann z.B. rastend mit dem Instrumentenkörper verbunden sein. Die Elektrode ist beispielsweise mit hochfrequentem elektrischem Strom gespeist und wirkt unter Ausbildung eines elektrischen Funkens auf das Gewebe ein.

In dem Instrumentenkörper oder einem mit diesem verbindbaren Element ist mindestens ein Fluidkanal vorgesehen, der an dem distalen Ende des Körpers oder des Elements eine Öffnung aufweist. Diese Öffnung kann insbesondere zur Absaugung von am Operationsort entstehenden Gasen, Dämpfen, Rauch, Gewebepartikeln oder dergleichen dienen. Ein solches den Absaugkanal aufweisendes Element kann auch die Elektrode und oder ein optisches Element aufweise, das von der Elektrode ausgehendes Licht aufnimmt und einer Lichtanalyseeinrichtung zuführt. Auch kann das optische Element dazu dienen, Licht an den Operationsort zu leiten.

Eine an dem Instrumentenkörper oder dem mit dem Instrumentenkörper verbundenen Element angeordnete Haube begrenzt dabei einen Raum, in den hinein und/oder durch den hindurch sich die Elektrode erstreckt und in dem die Öffnung des Kanals angeordnet ist. Der die Elektrode umgebende Raum kanalisiert vom Operationsort, d.h. von der Elektrode abgehende Emissionen und führt diese in den Fluidkanal. Diese Strömungsleitfunktion wird erreicht, indem die Haube über die distale Stirnfläche des Instrumentenkörpers oder des Elements vorsteht und so den genannten Raum bildet.

Das Instrument weist außerdem ein optisches Element auf, das an dem distalen Ende des Instrumentenkörpers oder des mit diesem verbindbaren Elements an einem Lichtübertrittsfenster angeordnet ist. Das Lichtübertrittsfenster kann eine Öffnung sein, durch die sich ein Sichtkegel des optischen Elements erstreckt. Das Lichtübertrittsfenster kann auch durch die distale Endfläche des optischen Elements selbst gebildet sein.

Im Betrieb können insbesondere durch die Wirkung der Absaugung von Gasen, Dämpfen, Rauch und/oder Gewebepartikeln, die am Operationsort entstehen, in dem von der Haube umgebenen Raum, insbesondere vor dem Lichtübertrittsfenster Ablagerungen entstehen, beispielsweise in Form von Gewebepartikelansammlungen. Solche Ablagerungen können den Lichtübertritt durch das Lichtübertrittsfenster behindern, und so die Funktionalität des Instruments einschränken.

Bei dem erfindungsgemäßen Instrument ist die Haube an dem Instrumentenkörper oder dem daran befestigten Element so beweglich angeordnet, dass sie in einer ersten Position den gewünschten zur Absaugung von Emissionen des Operationsorts nötigen Raum begrenzt und in einer zweiten Position das Lichtübertrittsfenster freigibt. In der ersten Position überragt die Haube das Lichtübertrittsfenster in distaler Richtung und versperrt so einen seitliche Zugang zu dem Lichtübertrittsfenster. Unter "seitlichem Zugang" wird ein Zugang quer zu der sich von proximal nach distal erstreckenden Längsrichtung verstanden. In der zweiten Position überragt die Haube das Lichtübertrittsfenster in distaler Richtung nicht oder nur so wenig, dass der seitliche Zugang zu dem Lichtübertrittsfenster frei ist. So kann das Instrument auf einfache Weise gereinigt werden. Es wird dazu lediglich die Haube von ihrer ersten in ihre zweite Position überführt. Vorhandene Partikel können dann einfach zum Beispiel mittels eines Tuchs entfernt werden. Dabei ist auch ausgeschlossen, dass etwaige Partikel und sonstige Ablagerungen lediglich verschmiert oder verschleppt werden. Die Handhabung ist einfach und intuitiv.

Der Fluidkanal ist, wie erwähnt, vorzugsweise ein Absaugkanal. Dazu kann das Instrument an seinem proximalen Ende mit einer Anschlussvorrichtung versehen sein, sodass der Fluidkanal an eine Absaugeinrichtung anschließbar ist. Zumindest optional kann dieser Kanal oder ein weiterer (zweiter) Fluidkanal als Spülfluidkanal genutzt werden. Wenn dies der Fall ist, kann der zweite Kanal an dem proximalen Ende des Instruments oder einer Anschlussleitung des Instruments über eine entsprechende Anschlusseinrichtung mit einer Spülfluidquelle verbindbar sein. Das Spülfluid kann ein gasförmiges Fluid wie z.B. Luft, Kohlendioxyd, Argon, Stickstoff oder irgendein anderes Gas sein. Das Spülfluid kann auch eine Flüssigkeit sein, wie z.B. wässrige NaCl-Lösung. Der zweite Kanal oder ein weiterer Kanal kann auch dazu dienen, der Elektrode Gas (z.B. Argon) zuzuführen beispielsweise um ein Plasma zu erzeugen.

Die zur Absaugung dienende Öffnung, die Elektrode und das Lichtübertrittsfenster sind vorzugsweise zueinander benachbart angeordnet und weiter vorzugsweise von der Haube umgeben. Somit sind die der Absaugung dienende Öffnung und das Lichtübertrittsfenster gemeinsam in oder an dem von der Elektrode durchquerten Raum zueinander benachbart angeordnet. Die Elektrode kann dabei in distaler Richtung aus der Haube herausstehend angeordnet sein. Die Haube umgrenzt dann eine distale Saugöffnung, mit der von der Elektrode bei der Gewebebehandlung entstehende Emissionen angesaugt und auf die Öffnung des Fluidkanals konzentriert werden.

Die Haube kann einen ringsum durchgehenden Rand aufweisen und in Längsrichtung, d.h. zwischen einer distalen Position und einer proximalen Position beweglich angeordnet sein. Dabei können Mittel vorgesehen sein, um der Haube einen translatorischen Bewegungsweg vorzugeben. Es ist aber auch möglich, Mittel vorzusehen, mittels derer die Haube auf einen schraubenförmigen Weg zwischen einer distalen ersten Position und einer proximalen zweiten Position bewegbar ist. Die Haube kann hohlzylindrisch oder hohlkegelförmig ausgebildet sein. Damit ist auch der von der Haube umgrenzte Raum zylindrisch oder kegelstumpfförmig ausgebildet.

Es können Mittel vorgesehen sein, um die Haube in der ersten Position und(oder der zweiten Position zu halten. Dies erleichtert die Handhabung. Solche Mittel können Rastmittel, Klemmmittel oder Ähnliches sein.

In einer alternativen Ausführungsform weist der Instrumentenkörper einen schalenförmigen Vorsprung auf und die Haube ist als ergänzende Halbschale ausgebildet. Der schalenförmige Vorsprung des Instrumentenkörpers und die Haube sind in diesem Fall sich jeweils nur teilweise um den Raum herum erstreckend ausgebildet. In der ersten Position bilden der Vorsprung des Instruments und die Haube eine ringförmige Umschließung des Raums, während die Haube in ihrer zweiten Position mit dem schalenförmigen Vorsprung des Instrumentenkörpers deckend angeordnet ist und somit den Raum zumindest nach einer Seite hin seitlich freigibt, sodass das Lichtübertrittsfenster zugänglich ist. Insoweit weist der Instrumentenkörper ein Reinigungsfenster auf, das von der Haube freigegeben oder verschlossen werden kann.

Weitere Einzelheiten vorteilhafter Ausführungsformen der Erfindung sind Gegenstand der Zeichnung und der zugehörigen Beschreibung. In der Zeichnung zeigen:
Figur 1 ein Instrument und ein zugehöriges Gerät zur Versorgung des Instruments, in schematisierter Darstellung,
Figur 2 das distale Ende des Instruments nach Figur 1, in einer ersten Ausführungsform und in schematisierter perspektivischer Darstellung,
Figur 3 das distale Ende des Instruments nach Figur 2 mit seiner Haube in erster Position, in längs geschnittener Darstellung,
Figur 4 das distale Ende des Instruments nach Figur 2 und 3 mit seiner Haube in zweiter Position (Freigabeposition) ,
Figur 5 eine abgewandelte Ausführungsform des Instruments nach den Figuren 1 bis 3 in längs geschnittener Darstellung,
Figur 6 eine weiter abgewandelte Ausführungsform des Instruments mit drehbar gelagerter Haube mit Haube in Schließstellung, in schematisierter perspektivischer Darstellung,
Figur 7 das distale Ende des Instruments nach Figur 6 mit Haube in Freigabeposition, in schematisierter perspektivischer Darstellung.

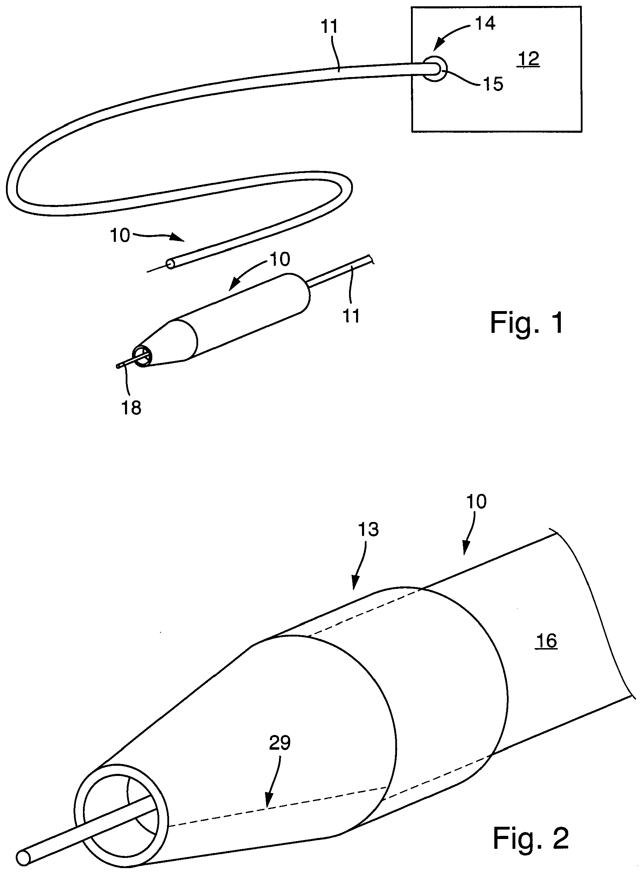

In Figur 1 ist ein erfindungsgemäßes Instrument 10 veranschaulicht, das mittels einer zugehörigen Leitung 11 an ein speisendes Gerät 12 angeschlossen ist. Das Instrument 10 kann als Sonde für den endoskopischen Einsatz, als laparoskopisches Instrument oder als Instrument für den offenchirurgischen Einsatz ausgebildet sein. Ein solches für den offenchirurgischen Einsatz vorgesehenes Instrument 10' ist in Figur 1 gesondert veranschaulicht. Die nachfolgend erläuterten Merkmale und Prinzipien gelten für die Instrumente 10, 10' gleichermaßen, unabhängig von ihrer sonstigen Ausbildung, insbesondere unabhängig davon, ob sie für den offenchirurgischen, den laparoskopischen oder den endoskopischen Einsatz ausgebildet ist.

Das Instrument 10, 10' weist ein in Figur 2 gesondert veranschaulichtes distales Ende 13 und ein proximales Ende 14 auf, das mit einem Instrumentenstecker 15 versehen sein kann, über den das Instrument 10, 10' mit dem Gerät 12 verbunden ist. Das Gerät 12 enthält alle zum Betrieb des Instruments 10, 10' erforderlichen Elemente und Baugruppen.

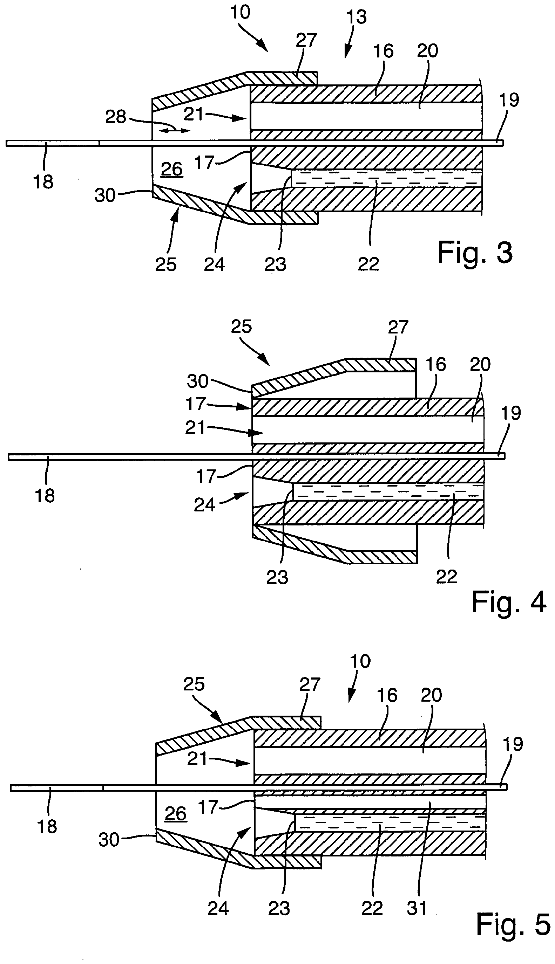

Zu dem Instrument 10 gehört ein Instrumentenkörper 16 (Figur 2, siehe auch Figur 3), von dessen distaler Stirnseite 17 wenigstens eine Elektrode 18 weg ragt. Die Elektrode kann eine Nadelform, Spatelform oder irgendeine andere für den operativen Zweck geeignete Form aufweisen. Zur Bestromung ist die Elektrode 18 mittels eines sich durch die Leitung 11 erstreckenden elektrischen Leiters 19 und über den Instrumentenstecker 15 mit dem Gerät 12 verbunden. Die Elektrode 18 wird von dem Gerät 12 über den Leiter 19 mit elektrischer Spannung und Strom. beispielsweise hochfrequentem Strom versorgt. Die Spannung ist dabei vorzugsweise so hoch dass zwischen der Elektrode 18 und in der Nähe befindlichem biologischem Gewebe ein Funken erzeugt werden kann.

Durch den Instrumentenkörper 16 erstreckt sich ein Fluidkanal 29, der an einer an der distalen Stirnseite 17 mündenden Öffnung 21 endet. Der Kanal 20 erstreckt sich durch den Instrumentenkörper 16 und die Leitung 11 zu dem Instrumentenstecker 15. In dem Gerät 12 kann eine Absaugeinrichtung vorgesehen sein, die fortwährend oder bedarfsweise einen in proximaler Richtung gerichteten Gasstrom und somit in der Öffnung 21 einen Saugeffekt erzeugt.

Zu dem Instrument 10 gehört außerdem ein optisches Element 22, das in Figur 3 beispielhaft als Lichtleitfaser veranschaulicht ist. Anstelle einer einzelnen Lichtleitfaser kann das optische Element 22 jedoch auch aus einem Bündel mehrerer Lichtleitfasern bestehen und/oder weitere optische Elemente, wie beispielsweise Linsen, Spiegel oder dergleichen, umfassen. Das optische Element 2 weist an seinem distalen Ende eine Lichtübertrittsfläche 23 auf, die Licht aussenden und/oder Licht empfangen kann. Das optische Element 22 ist beispielsweise in Gestalt einer Lichtleitfaser, die sich bis zu dem Gerätestecker 15 erstreckt, dazu eingerichtet, an der Lichtübertrittsfläche 23 aufgenommenes Licht an das Gerät 12 zu liefern.

Die Lichtübertrittsfläche 23 kann bündig an der distalen Stirnseite 17 des Instrumentenkörpers 16 angeordnet oder, wie in Figur 3 dargestellt, gegen diese Fläche zurückgesetzt angeordnet sein. Dazu ist in der distalen Stirnsete eine entsprechende Öffnung ausgebildet, die ein Lichtübertrittsfenster 24 bildet. Ist die Lichtübertrittsfläche 23 bündig an der distalen Stirnseite 17 angeordnet, bildet die Lichtübertrittsfläche 24 selbst das Lichtübertrittsfenster 24.

An dem distalen Ende 13 des Instruments 10 ist außerdem eine Haube 25 angeordnet, die einen an die distale Stirnseite 17 des Instrumentenkörpers 16 angrenzenden Raum 26 umgrenzt. Durch diesen Raum 26 erstreckt sich die Elektrode 18. Die Haube 25 weist zum Beispiel eine hohle kegelstumpfförmige Gestalt auf. Sie kann außerdem einen hohlzylindrischen Abschnitt 27 aufweisen, der auf der beispielsweise zylindrischen Außenfläche des Instrumentenkörpers 16 sitzt. Die Haube 25 dient zu Verbesserung des Absaugeffekts und zur Verteilung der von dem Fluidkanal 29 ausgehenden Sogwirkung um die gesamte Elektrode 18 herum.

Die Haube 25 ist in Axialrichtung 28 zwischen zwei Positionen hin und her bewegbar angeordnet. Figur 3 veranschaulicht die Haube 25 in einer ersten Position, in der das Instrument 10 zur Behandlung von Gewebe einsetzbar ist und in der die Haube 25 die gewünschte Leitwirkung für die Absaugströmung aufweist. Figur 4 veranschaulicht die Haube 25 in ihrer zweiten axial in Proximalrichtung zurückgezogenen Position, in der sie die distale Stirnfläche 17 des Instrumentenkörpers 16 weitgehend freigibt. Um ein solches Zurückziehen der Haube 25 zu ermöglichen kann die Haube 25 z.B. entlang einer Mantellinie eine in Figur 2 gestrichelt veranschaulichte Fuge 29 aufweisen an der die Haube 25, wenn sie in die Rückzugsposition gemäß Figur 4 überführt wird, sich federnd aufweiten kann. Die Federwirkung der Haube 25 kann dazu beitragen, die Haube 25 im Einsatz in ihrer ersten Position zu halten und die Haube 25 auf diese Position hin vorzuspannen.

Das insoweit beschriebene Instrument 10 arbeitet wie folgt:
Zur Behandlung eines Patienten wird das Instrument 10 oder 10' mit seiner Elektrode 18 an den zu behandelnden Gewebebereich angenähert und der Generator 12 aktiviert. Die Elektrode 18 wird mit hochfrequenter Wechselspannung beaufschlagt, sodass zwischen dem biologischen Gewebe und der Elektrode 18 ein Funken entsteht. Dabei durch Einwirkung des Funkens auf das Gewebe entstehender Dampf, Rauch, auch Gewebebruchstücke und dergleichen, werden über den Fluidkanal 20 abgesaugt. Dabei verteilt die Haube 25, die sich in der in Figur 3 veranschaulichten Position befindet, die Sogwirkung gleichmäßig um die Elektrode 18, sodass ein guter Absaugeffekt gegeben ist.

Über das optische Element 22 wird von dem Funken ausgehendes Licht aufgenommen. Dieses tritt über das Lichtübertrittsfenster 24 und die Lichtübertrittsfläche 23 in das optische Element 22 ein und wird von diesem dem Gerät 12 zugeleitet. Dort kann eine Analyse des empfangenen Lichts vorgenommen werden, um auf Merkmale des Gewebes die Qualität des Funkens oder andere Gegebenheiten zu schließen.

Durch die chirurgische Einwirkung auf das Gewebe und die Absaugwirkung ist damit zu rechnen, dass Partikel, Gewebebruchstücke, Flüssigkeitstropfen und dergleichen in den Raum 26 eingesaugt und dort auch an dem Lichtübertrittsfenster 24 niedergeschlagen werden. Ist dies der Fall, wird der optische Weg zwischen der Elektrode und dem Lichtübertrittsfenster 24 zunehmend versperrt.

Das erfindungsgemäße Instrument 10 ermöglicht eine einfache Reinigung, indem die Haube 25 aus ihrer in Figur 3 veranschaulichten Position in eine zweite, in Figur 4 veranschaulichte Position überführt wird. Während der Rand 30 der Haube 25 in Arbeitsposition gemäß Figur 3 in distaler Richtung weit von der Stirnfläche 17 entfernt steht und die Haube 25 den seitlichen Zugang zu dem Lichtübertrittsfenster 24 verdeckt, ist der Rand 30 in der zweiten Position der Haube 25 bündig zu der oder zumindest nahe bei der Stirnfläche 17. In dieser Position ist das Lichtübertrittsfenster 24 seitlich zugänglich und kann beispielsweise mittels eines Lappens leicht gereinigt werden, sodass das Instrument 13 wieder betriebsbereit ist. Um vollständige Betriebsfähigkeit herzustellen, wird die Haube 25 aus der in Figur 4 veranschaulichten Position zurück in die Position gemäß Figur 3 überführt.

Es können nicht weiter veranschaulichte Rast- oder Klemmmittel vorgesehen sein, um die Haube 25 in Arbeitsposition gemäß Figur 3 und/oder in rückgezogener Position, d.h. Reinigungsposition gemäß Figur 4 zu halten.

Die vorstehende Beschreibung ist davon ausgegangen, dass die Haube 25 hohlkegelstumpfförmig ausgebildet ist. Es ist jedoch auch möglich, andere Formen zu nutzen, beispielsweise eine Rotationshyperboloidform, eine Hohlzylinderform oder dergleichen. Außerdem kann auf die Fuge 29 verzichtet werden, wenn die Haube 25 aus einem ausreichend elastischen Material oder aus mehreren Lamellen besteht, die voneinander spreizbar sind und von dem Abschnitt 27 in Distalrichtung abstehen.

Eine weitere Ausführungsmöglichkeit veranschaulicht Figur 5. Für diese Ausführungsform gilt die vorige Beschreibung uneingeschränkt entsprechend. Außerdem gilt, dass zusätzlich zu dem Fluidkanal 20 ein zweiter Fluidkanal 31 vorgesehen sein kann, der sich von dem Instrumentenstecker 15 bis zu der distalen Stirnfläche 17 des Instrumentenkörpers 16 erstreckt. Über den Fluidkanal 31 kann ein Spülfluid zu dem optischen Element 22 und insbesondere dessen Lichtübertrittsfläche 23 geführt werden. Das Spülfluid kann ein Gas oder auch eine Flüssigkeit insbesondere NaCl-Wasser-Lösung sein. Das Spülfluid kann von dem Gerät 12 geliefert werden, um die Lichtübertrittsfläche 23 und/oder das Lichtübertrittsfenster 24 sauber zu halten oder zu reinigen. Die Lieferung des Spülfluids kann manuell durch entsprechende Betätigungselemente ausgelöst werden oder automatisiert ablaufen, beispielsweise jeweils nach dem Ende der Aktivierung der Elektrode 18.

Auch bei dieser Ausführungsform des Instruments 10 ist die Haube 25 zwischen zwei Positionen verstellbar, nämlich der in Figur 5 dargestellten Arbeitsposition und einer Reinigungsposition gemäß Figur 4. Die Haube 25 ist verschiebbar angeordnet, um seitlichen Zugang zu dem Lichtübertrittsfenster 24 zu eine manuelle Reinigung desselben zu ermöglichen.

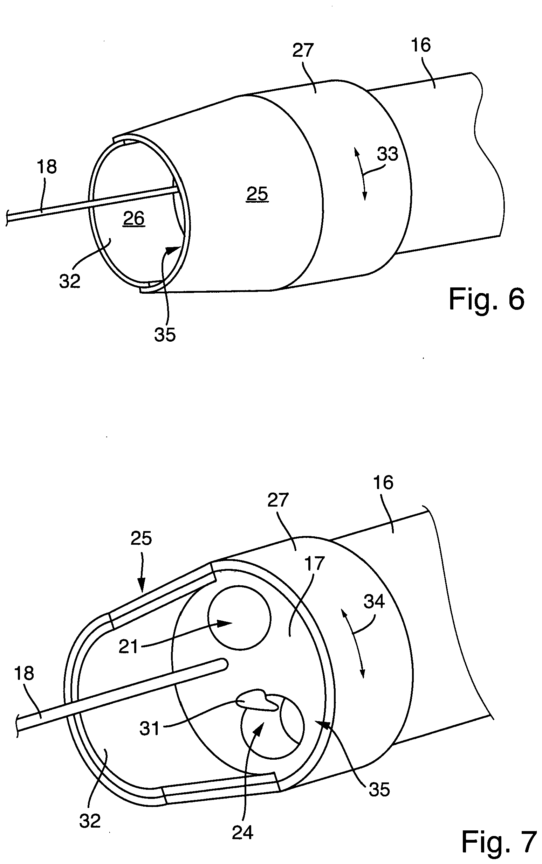

Die vorstehende Beschreibung setzt eine axiale Bewegungsmöglichkeit der Haube 25 voraus. Dies kann eine geradlinige translatorische Bewegung zwischen zwei Endpositionen der Haube 25 sein. Der Weg kann auch schraubenartig gewunden sein. Eine weitere Variante zeigen die Figuren 6 und 7:

Bei der Ausführungsform des Instruments 10 nach Figur 6 weist der Instrumentenkörper 16 einen schalenartigen Fortsatz 32 auf, der den Raum 26 ungefähr um die Hälfte seines Umfangs begrenzt und an seiner diametral gegenüberliegenden Seite ein Reinigungsfenster 35c frei lässt. Die andere Hälfte des Umfangs wird von der hier schalenförmigen Haube 25 umgrenzt, die so das Reinigungsfenster 35 schließt. Der Fortsatz 32 und die Haube 25 ergänzen sich, wie Figur 6 zeigt, zusammen zu einer Hohlkegelstumpfform und umgrenzen somit den Raum 26 gemeinsam. Entsprechend der vorigen Beschreibung kann anstelle der Hohlkegelstumpfform auch jede andere Form gewählt werden die die von der Öffnung 21 ausgehende Sogwirkung wie gewünscht rings um die Elektrode 18 herum kanalisiert.

Bei der Ausführungsform nach den Figuren 6 und 7 ist der Abschnitt 27 der Haube 25 auf dem Instrumentenkörper 6, wie Pfeile 33, 34 andeuten, drehbeweglich gelagert. In einer ersten Arbeitsposition gemäß Figur 6 ergänzen sich der Fortsatz 32 und die Haube 25 zu einer um den Raum 26 ringsum geschlossenen Struktur. In der zweiten (Dreh-) Position der Haube 25 nach Figur 7 stehen der Fortsatz 32 und die Haube 25 ungefähr deckungsgleich, sodass das Reinigungsfenster 35 offen steht und die distale Stirnfläche 17 des Instrumentenkörpers 16 zumindest weitgehend frei liegt. Insbesondere ist das Lichtübertrittsfenster 24 von der Seite her frei zugänglich, sodass dort niedergeschlagene Gewebepartikel oder sonstige Ablagerungen leicht manuell entfernt werden können.

Die Elektrode 18 und/oder der Fluidkanal 20 und/oder der Fluidkanal 31 und/oder das optische Element 22 und/oder die Haube 25 können wie beschrieben jeweils an dem Instrumentenkörper 16 oder alternativ an einem gesonderten, mit dem Instrumentenkörper verbundenen, insbesondere lösbar verbundenen Element angeordnet sein. Das Element kann z.B. mittels einer Steckverbindung oder einer Rastverbindung mit dem Instrumentenkörper 16 verbunden sein.

Das erfindungsgemäße Instrument 10 weist eine Haube 25 auf, die einen Strömungsraum 26 umschließt. Dieser dient der Absaugung von Emissionen, die durch Einwirkung einer hochfrequenzspannungsbeaufschlagten Elektrode 18 auf biologisches Gewebe entstehen. Zur Beseitigung von Gewebeniederschlag und sonstigem Verstopfungen aus den Strömungsraum 26 wird dieser von einer beweglichen Haube 25 begrenzt, die zu Reinigungszwecken aus ihrer Arbeitsposition heraus in eine Reinigungsposition überführbar ist.

### Bezugszeichen:

- 10, 10': Instrument
- 11: Leitung
- 12: Gerät
- 13: distales Ende des Instruments 10, 10'
- 14: proximales Ende
- 15: Instrumentenstecker
- 16: Instrumentenkörper
- 17: distale Stirnseite des Instrumentenkörpers 16
- 18: Elektrode
- 19: elektrischer Leiter
- 20: Fluidkanal
- 21: Öffnung
- 22: optisches Element
- 23: Lichtübertrittsfläche
- 24: Lichtübertrittsfenster
- 25: Haube
- 26: Raum
- 27: Abschnitt der Haube 25
- 28: Axialrichtung
- 29: Fuge
- 30: Rand
- 31: Fluidkanal
- 32: Fortsatz
- 33, 34: Pfeile
- 35: Reinigungsfenster

## Patentansprüche

1. Instrument (10) zur elektrochirurgischen Behandlung menschlicher oder tierischer Patienten,
mit einem Instrumentenkörper (16), der ein distales Ende (13) und mindestens einen Fluidkanal (20) aufweist, der sich von einer an dem distalen Ende (13) angeordneten Öffnung (21) in proximaler Richtung durch den Instrumentenkörper (16) oder ein an dem Instrumentenkörper angebrachtes Element erstreckt,
mit einer Elektrode (18), die an dem distalen Ende (13) des Instrumentenkörpers (16) oder einem an dem Instrumentenkörper angebrachten Element gehalten ist,
mit einem optischen Element (22), das an dem distalen Ende (13) an einem Lichtübertrittsfenster (24) angeordnet ist,
mit einer Haube (25), die einen Raum (26) begrenzt, durch den sich die Elektrode (18) erstreckt und in dem die Öffnung (21) und das Lichtübertrittsfenster (24) angeordnet sind, wobei die Haube (25) zwischen mindestens einer ersten Position, in der sie das Lichtübertrittsfenster (24) verdeckt, und einer zweiten Position hin und her beweglich gelagert ist, in der sie das Lichtübertrittsfenster (24) freigibt.

2. Instrument nach Anspruch 1, **dadurch gekennzeichnet, dass** der Fluidkanal (20) an eine Absaugeeinrichtung anschließbar ist und das die Öffnung (21) eine Absaugöffnung ist.

3. Instrument nach einem der vorstehenden Ansprüche, **dadurch gekennzeichnet, dass** die Elektrode (18) mit einem sich durch das Instrument (10) in proximaler Richtung erstreckenden Leiter (19) an einen elektrischen Generator (12) anschließbar ist.

4. Instrument nach einem der vorstehenden Ansprüche, **dadurch gekennzeichnet, dass** die Öffnung (21), die Elektrode (18) und das Lichtübertrittsfenster (24) zueinander benachbart an einer distalen Stirnfläche des Instrumentenkörpers (16) angeordnet sind.

5. Instrument nach einem der vorstehenden Ansprüche, **dadurch gekennzeichnet, dass** die Elektrode (18) in distaler Richtung aus der Haube (25) herausstehend angeordnet ist.

6. Instrument nach einem der vorstehenden Ansprüche, **dadurch gekennzeichnet, dass** die Haube (25) eine distale Saugöffnung aufweist.

7. Instrument nach Anspruch 7, **dadurch gekennzeichnet, dass** die Saugöffnung einen ringsum durchgehenden Rand (30) aufweist.

8. Instrument nach einem der vorstehenden Ansprüche, **dadurch gekennzeichnet, dass** die Haube (25) zwischen einer distalen und einer proximalen Position beweglich angeordnet ist.

9. Instrument nach Anspruch 8, **dadurch gekennzeichnet, dass** die Haube (25) translatorisch beweglich angeordnet ist.

10. Instrument nach einem der vorstehenden Ansprüche, **dadurch gekennzeichnet, dass** die Haube (25) hohlzylindrisch oder hohlkegelstumpfförmig ausgebildet ist.

11. Instrument nach einem der vorstehenden Ansprüche, **dadurch gekennzeichnet, dass** die Haube (25) und ein Fortsatz (32) des Instrumentenkörpers (16) sich jeweils nur teilweise um den Raum (26) erstreckend angeordnet sind.

12. Instrument nach Anspruch 11, **dadurch gekennzeichnet, dass** die Haube (25) auf dem Instrumentenkörper (16) drehbar gelagert ist.

13. Instrument nach einem der vorstehenden Ansprüche, **dadurch gekennzeichnet, dass** der Instrumentenkörper (16) ein Reinigungsfenster (35) aufweist.

14. Instrument nach den Ansprüchen 11, 12 und 13, **dadurch gekennzeichnet, dass** die Haube (25) in ihrer ersten Position das Reinigungsfenster (35) überdeckend und in ihrer zweiten Position das Reinigungsfenster freigebend angeordnet ist.

15. Instrument nach einem der vorstehenden Ansprüche, **dadurch gekennzeichnet, dass** der Instrumentenkörper (16) einen zweiten Kanal (31) aufweist.
